# EUROPEAN PATENT APPLICATION

(11) **EP 3 878 372 A1**
(43) Date of publication of application: **15.09.2021**
(21) Application number: 20382178.0
(22) Date of filing: 11.03.2020
(51) Int. Cl.: A61B 8/10, A61B 3/16

(54) **METHOD AND SYSTEM FOR SCREENING OCULAR TISSUE ABNORMALITY THROUGH DEFORMATION OF THE OCULAR TISSUE**

(71) Applicant: National University of Ireland, Galway, H91 TK33 Galway (IE); Consejo Superior De Investigaciones Científicas, 28006 Madrid (ES); The University Of Liverpool, Liverpool L69 7ZX (GB)
(72) Inventor: Nolan, Andrew, Galway, Ireland, H91 TK33 (IE); McAuley, Ryan, Galway, Ireland, H91 TK33 (IE); Alexandrov, Sergey, Galway, Ireland, H91 TK33 (IE); Leahy, Martin, Galway, Ireland, H91 TK33 (IE); Birkenfeld, Judith, 28006 Madrid (ES); Curatolo, Andrea, 28006 Madrid (ES); Marcos, Susana, 28006 Madrid (ES); Elsheikh, Ahmed, Liverpool, United Kingdom, L69 7ZX (GB); Abass, Ahmed, Liverpool, United Kingdom, L69 7ZX (GB); Eliasy, Ashkan, Liverpool, United Kingdom, L69 7ZX (GB)
(74) Representative: Balder IP Law, S.L.

(57) **Abstract**

The invention relates to a method for screening a biomechanical abnormality of ocular tissue through deformation of the ocular tissue, the method comprising:
- generating an acoustic stimulus for delivery onto the ocular tissue in a collinear manner with an optical axis of an imaging device for producing a vibration of the ocular tissue;
- capturing with the imaging device a plurality of locations of the ocular tissue;
- processing the plurality of locations to screen the biomechanical abnormality of ocular tissue from an asymmetry in a frequency response of the vibration of the ocular tissue as measured at the plurality of locations.

The invention also relates to a system for screening a biomechanical abnormality of ocular tissue (C).

## Description

### TECHNICAL FIELD

The present invention generally relates to the field of ophthalmology. More specifically, the present invention relates to quantifying and/or detecting biomechanics of ocular tissue, particularly, the cornea, by means of producing non-contact deformation in the ocular tissue.

### STATE OF THE ART

Keratoconus is a form of ectasia, a progressive disease of the eye, resulting in localized thinning and reshaping of the cornea and localized change in corneal biomechanics. This can adversely affect vision, resulting in near-sightedness, astigmatism and light sensitivity. Keratoconus affects approximately 1% people.

For a number of reasons, from early diagnosis to prevention of post-LASIK ectasia, the ability to screen for corneal abnormalities is important.

Current solutions to this problem include the use of applanation tonometry information, from a device, such as the Corvis ST (Oculus Optikgeräte GmbH, Wetzlar, Germany) to reconstruct the biomechanics of the cornea. However, the use of such a device for screening purposes is commercially unfeasible.

Some other solutions involve the application of a large air puff or a contact device to the eye to estimate the force needed to flatten (applanate) the central cornea. This is an uncomfortable process (normal stimulation of the cornea can be difficult), which may lead to problems in repeatability and interpretation of results. Also, calibration of the air puff and accurate information about the force applied to the cornea can be complicated.

Therefore, there is a need for a non-invasive approach for screening abnormality of ocular tissue (such as the cornea), which does not pose the above-mentioned drawbacks.

### DESCRIPTION OF THE INVENTION

The present invention refers to a method and system for screening ocular tissue abnormality through deformation of the ocular tissue according to claims 1 and 10, respectively. Preferred embodiments of the method and system are defined in the dependent claims.

The present disclosure intends to solve the shortcomings of prior-art devices and methods by providing a method and a system for screening abnormality of ocular tissue, such as corneal tissue, based on the response (frequency and amplitude) of the cornea to a dynamic stimulation using vibrography.

According to the system of the present disclosure, an imaging device is combined with an acoustic excitation module which are mounted in coalignment. The coalignment of the acoustic stimulation and the imaging channel allows for simultaneous stimulation and measurement of multiple points distributed throughout the surface of the ocular tissue, more particularly the corneal apex. This configuration greatly simplifies the analysis and interpretation of the observed corneal frequency response in order to detect resonance frequencies, which may serve as a biomarker for early detection of corneal diseases.

The proposed solution has the advantages of being a low-cost solution, and also, precision of the coalignment between stimulation and measurement can be easily achieved thereby removing complications due to misalignment between stimulation and imaging. Vibration amplitudes can be in the orders of micrometres or nanometres, having the advantage that measurements are less straining in the patients' eye.

In some embodiments, the ocular tissue is excited over a large bandwidth of frequencies, and the frequency response and vibrational modes of the ocular tissue, which are dependent on the ocular tissue biomechanical properties, can be quantified and used to probe changes in the cornea due to disease.

Asymmetries are analysed within the excited resonance modes of the ocular tissue; deformation asymmetries are not analysed from a single deformation event, but within a wave.

The acoustic waves incident on the ocular tissue are produced by an acoustic excitation module (such as a speaker, voice coil etc.) which is being fed an oscillating voltage signal.

The acoustic excitation module can exhibit a resonance effect so the amplitude of the stimulation varies greatly across frequencies. In certain embodiments, the method further comprises characterising the frequency response of the acoustic stimulus. This characterisation of the acoustic excitation module can be measured by mounting a microphone in front of the acoustic excitation module, preferably near the sample.

In certain embodiments, the method further comprises pre-compensation, wherein the voltage signal passed to speaker is adjusted to ensure the signal produced by the speaker has a constant amplitude at all frequencies.

In some embodiments, in order to correct the measured corneal frequency response for the acoustic excitation module, the frequency response of the ocular tissue can be post-compensated, where the observed frequency response of the ocular tissue is adjusted according to the acoustic stimulus response.

In some embodiments the ocular tissue is the cornea, which allows for screening corneal biomechanical abnormality; but it could also be the sclera.

In some embodiments, the method further comprises obtaining intraocular pressure of the ocular tissue from a resonance mode analysis of the vibration of the ocular tissue independently from biomechanics.

The above method in any of its embodiments can be carried out using optical coherence tomography.

In any of the previous embodiments, the imaging device can be an optical coherence tomography imaging system.

The present invention provides a system that measures localised asymmetries in the corneal vibrational frequency response (resonance modes) by means of acoustic excitation collinear with the imaging system.

The different aspects and embodiments of the invention defined in the foregoing can be combined with one another, as long as they are compatible with each other.

Additional advantages and features of the invention will become apparent from the detail description that follows and will be particularly pointed out in the appended claims.

### BRIEF DESCRIPTION OF THE DRAWINGS

To complete the description and in order to provide a better understanding of the invention, a set of drawings is provided. Said drawings form an integral part of the description and illustrate embodiments of the invention, which should not be interpreted as restricting the scope of the invention, but just as an example of how the invention can be carried out. The drawings comprise the following figures:
Figure 1 shows a schematic view of the system components according to a possible embodiment of the system of the invention.
Figure 2 shows a detailed scheme of a speaker of the system of Fig. 1, showing its centred hole.
Figure 3 shows two possible beams configurations of the swept-source OCT system.
Figure 4 shows a block diagram of the speaker pre-compensation process.
Figure 5 shows the waveform recorded by the microphone from the speaker.
Figure 6 shows the frequency response of the speaker.
Figures 7 and 8 show the frequency response of the speaker and the signal from the speaker measured by a microphone, after the pre-compensation process.
Figures 9-10 show the frequency sweep signal in the time and frequency domains, respectively.
Figures 11-12 show the sinc signal in the time and frequency domains, respectively.
Figure 13 shows the frequency response of cornea in response to uncompensated acoustic stimulation.
Figure 14 shows the frequency response of cornea in response to post-compensated acoustic stimulation.
Figure 15 shows the frequency response at varying positions in a porcine eye subjected to local crosslinking.
Figure 16 shows the peak amplitude minus the peak amplitude at corneal apex vs position relative to corneal apex, for the test of Fig. 15.
Figure 17 presents examples of the asymmetry observed in keratoconic corneas with different IOP and SSI (Stress-Strain Index, a measure of corneal stiffness).

### DETAILED DESCRIPTION OF EMBODIMENTS

The following description is not to be taken in a limiting sense but is given solely for the purpose of describing the broad principles of the invention. Embodiments of the invention will be described by way of example, with reference to the above-mentioned drawings.

The present disclosure proposes the use of vibrography to determine the presence of corneal abnormality. Vibrography is the process of stimulating an object at a given frequency or range of frequencies and observing its response.

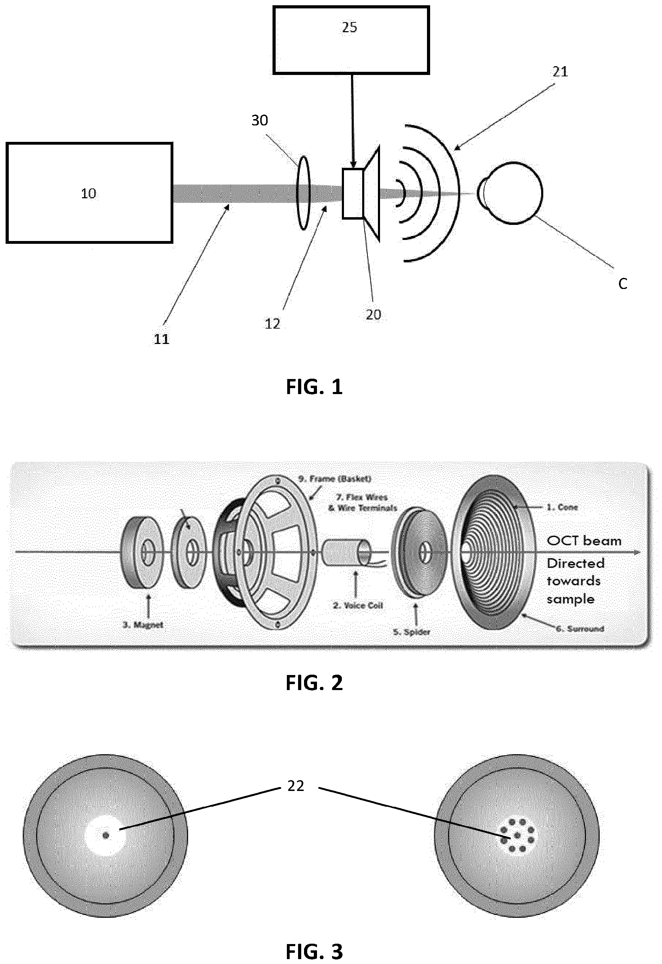

Figure 1 shows a schematic view of the system components according to a possible embodiment of the system of the invention. As shown, the system includes an imaging device formed by a swept-source OCT system 10 (also named ssOCT in the following), which is coupled with an acoustic stimulation module, in the present example in the form of a speaker 20 for inducing deformation into a sample, which in present example is an eye 1, more specifically, cornea of the eye.

The speaker 20 has a hole 22 in its centre (the hole is not visible in this Figure 1).

The ssOCT 10 provides light beam(s) 11 which pass through an objective lens 30, which focuses the beams 12 through the speaker 20 and onto the cornea C. The light beams 11, 12 are reflected from the sample back to the ssOCT 10.

The objective lens 30 may be part of the ssOCT 10 itself instead of being a separate component.

As shown in Figure 1, the speaker 20 and the ssOCT 10 and the speaker 20 are mounted in a collinear manner, allowing coalignment of the acoustic stimulation and an imaging channel (schematically represented by the scanning beams 11, 12) of the ssOCT 10 are aligned.

The ssOCT 10 employs a polygon scanner swept laser in the spectral range from 1.23 to 1.34 µm with a typical sweep rate of 45 kHz and an optical power of less than 10 mW on the sample. The axial resolution of the system is 15µm, and the axial field of view is 7.8mm.

The speaker 20 is fed by an oscillating voltage signal produced by a function generator 25, to produce acoustic waves 21 which are directed onto the cornea C. The oscillating voltage signal has a flat bandwidth. The function generator generates a frequency sweep from 0 to 1000 Hz with a peak to peak signal of 10 V with no dc voltage. The sweep duration was 1 s.

Figure 2 shows an exploded schematic view of a speaker 20 of the system of the invention including a cone 1 and surround 6, a voice coil 2, a magnet 3, a tope plate 4, a spider 5, and a frame or basket 7. To achieve the coaxial setup of the speaker and imaging system, a feature of this speaker is that a centred hole 22 has to be made through the full depth of the speaker 2, including the different components and also the pole cap and the dust cap of the cone 1 (the caps are not visible in Fig. 2).

The hole 22 has to be large enough in diameter to allow a light beam or multiple light beams (for the single and multi-beam setups shown in Fig. 3) to pass through the full length of the speaker, reflect from the sample and pass back through the centre of the speaker to the imaging system. Through this hole 22 the scanning beam 11, 12 from the ssOCT 10 can pass to reach the sample, in this example, the cornea (not shown in this fig. 2) and back to the ssOCT 10. As previously explained, this configuration allows for the coaxial alignment of the light beam/beams from the imaging system and acoustic waves from the speaker.

This figure 2 shows the speaker schematic for the single beam setup, but the principle is exactly the same for the multi-beam setup but with multiple beams passing through the centre of the speaker.

Beams are focused on the sample and so the objective lens 30 used needs to have a focal length which is larger than the depth of the speaker 20 and also reach the sample to be imaged.

Figure 3 shows a front view of the speaker 20 with two possible beams configurations passing through the hole 22: with a single beam (left image of Fig. 3) and a multi-beam configuration, of nine points (right image of Fig. 3).

In either configuration, the acoustic waves produced from the speaker in response to the voltage signal supplied by the computer/function generator are incident on the sample, in this case, the cornea C. The distance between speaker 20 and cornea C is 5-10 mm. These acoustic waves induce some motion, typically in the form of vibration, of the sample which is detected by the ssOCT from the reflected light beams.

In order that these acoustic waves have the same bandwidth as the voltage signal and also have a flat bandwidth, they are subjected to a pre-compensation process, in order to compensate for the effect of the own frequency response of the speaker.

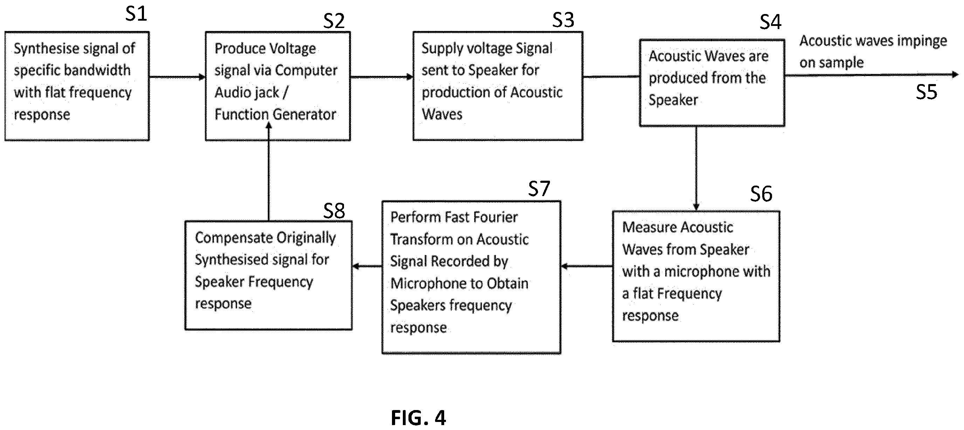

Figure 4 shows a block diagram of the generation of acoustic waves and the speaker pre-compensation process.

Firstly (step S1), a voltage signal is synthesized using signal processing programming, with a predefined amplitude, frequency bandwidth and flat frequency response. The voltage signal is produced from the computer or function generator 25 (step S2) and applied to the speaker 20 via an audio jack, USB, cables or similar (step S3) for producing acoustic waves (step S3). The acoustic waves are produced from the speaker in response to the voltage signal (step S4) and impinge on the sample to be measured (step S5).

The acoustic waves produced have the same bandwidth as the voltage signal, but a different frequency response. This is due to the nature of the speaker 20 which has a frequency response of its own.

There are several methods for characterising the speaker: measurement of diaphragm movement using OCT; measurement of impedance of speaker and measurement of sound produced by the speaker using a microphone. The former two methods are indirect measurements of the acoustic stimulation produced by the speaker. In the present case, the frequency response of the speaker is characterised and compensated using a microphone. The advantage is that it characterises the acoustic stimulation produced by the speaker directly, and can be examined at a position very similar to the location of the cornea during measurement.

This frequency response of the speaker can be pre-compensated for using a microphone as explained in the following paragraphs. It is to be noted that the frequency response of the microphone is observed to be flat, and thus, compensation for the response of the microphone is considered unnecessary.

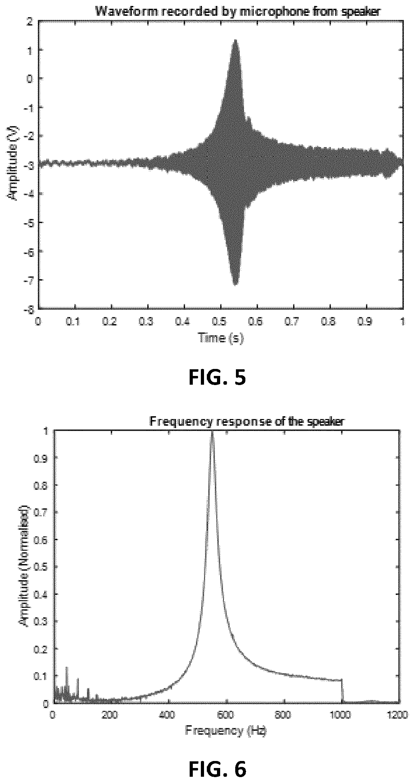

A microphone (not shown in the drawings) with a known, flat frequency response in the frequency range of interested is placed in front of the speaker, as near as possible to where the sample is placed. The acoustic signal from the speaker is recorded by the microphone (step S6) and shown in Figure 5.

A Fast Fourier Transform (FFT) is performed on this recorded acoustic signal which reveals the frequency response/content of the acoustic waves at the position where the microphone is, and so, representative of the position of the sample (step S7). This frequency response is shown in Figure 6, it is seen to be near constant with changing location and signal amplitude, and in this case having a clear resonance at around 550-570 Hz. In order to create a flat acoustic frequency sweep from this speaker, a linear frequency sweep signal that is the inverse of the speaker frequency response (shown in Figure 6) should be supplied to the speaker. Thus, the frequency response of the speaker is then normalised by the highest peak in the response. The originally synthesised flat voltage signal is normalised and divided by the normalised signal from step S6. This new compensated signal is then supplied to the speaker (step S8).

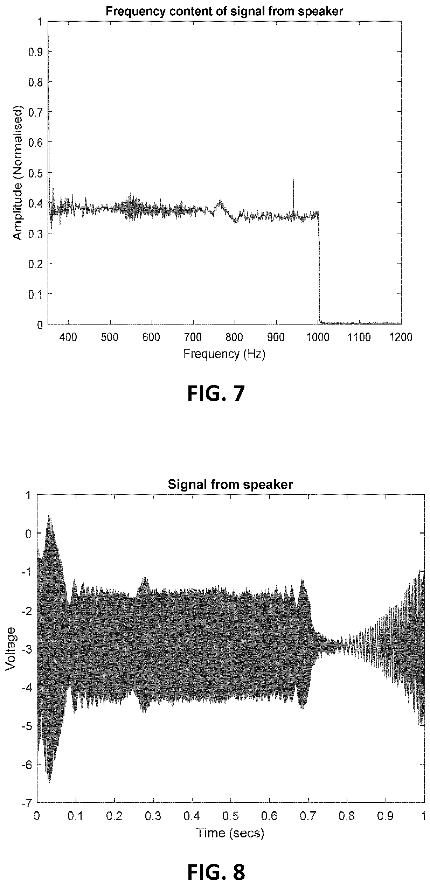

After this process, the acoustic signal produced by the speaker is pre-compensated and has a flat frequency response at the position of the microphone/sample, as shown in Figure 7. Figure 8 shows the signal from the speaker after this pre-compensation process as measured by the microphone.

For the acoustic waves to be impinged onto the sample, two stimulation regimes can be considered: a frequency sweep regime in which each frequency is probed individually and sequentially, and sinc regime where a range of frequencies are probed simultaneously using a sinc pulse.

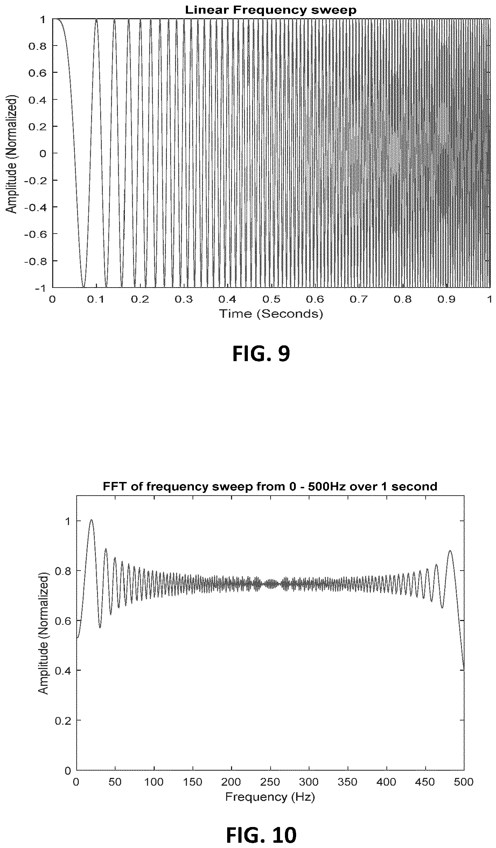

In the frequency sweep regime, the frequencies of interest are scanned individually and sequentially. Each frequency is sampled at the same amplitude for the same length of time. While this regime has the advantage of a higher amplitude at a given frequency, the Fourier Transform of the signal produces Fresnel ripples which can complicate the interpretation of the resultant corneal response. The frequency sweep signal is shown in Figures 9-10 in the time and frequency domains, respectively.

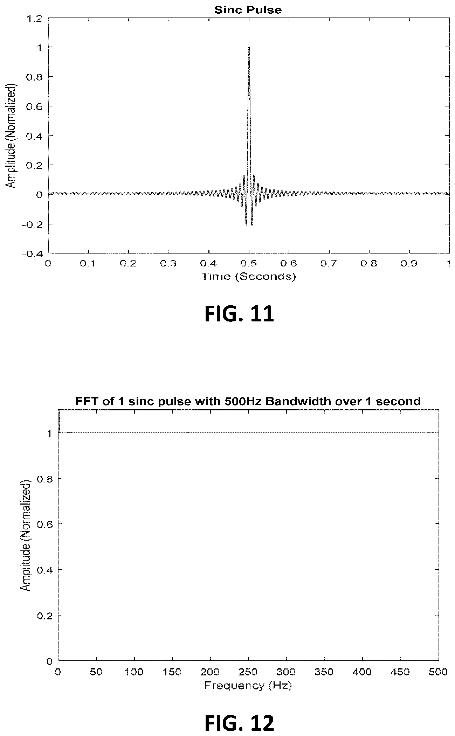

In the sinc regime, all frequencies of interest are sampled simultaneously using a sinc pulse. While this signal can probe all frequencies more quickly, there is a lower signal amplitude at a given frequency. The sinc signal is shown in Figures 11-12 in the time and frequency domains, respectively.

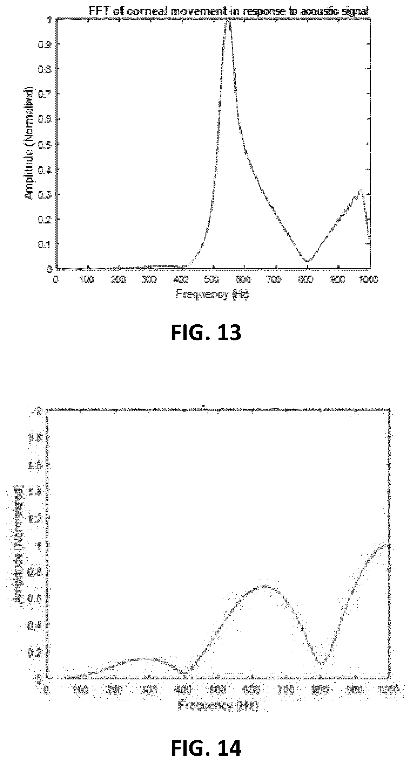

Another possibility for compensating the frequency response of the speaker is to post-compensate for it in the processing of the observed corneal frequency. In this case, the frequency response of the speaker is also characterised using a microphone (as in the pre-compensation process). As shown in Figure 6, the frequency response of the speaker has a clear resonance at around 550-570 Hz. If the signal from the speaker is used for stimulating the cornea, the corneal response (as can be seen in Figure 13) appears similar to the speaker response suggesting that the measured response is more related to the speaker than the cornea.

The frequency response of the speaker can be used to apply post-compensation as follows: the corneal movement signal spectrum, measured with the ss-OCT is divided by the frequency response of the speaker. It is actually quite a powerful, albeit simple technique as can be seen from the post-compensated corneal frequency response (cf. Figure 14) in comparison with the uncompensated frequency response of the cornea (cf. Figure 13).

Post-compensation can be used in correcting the measured corneal response for the speaker response; however, information about the absolute movement of the cornea under stimulation is lost under this post-compensation process. This loss of information does not happen when using pre-compensation, and the signal passed to the speaker is adapted to ensure the amplitude of the acoustic stimulation remains constant at all frequencies (a flat signal across the frequency range of interest is generated).

### Tests & results

Freshly enucleated porcine eyes were obtained from a slaughterhouse (Matadero Justino Gutiérrez, Laguna de Duero, Valladolid, Spain) prior to scalding, and used within 48 hours postmortem. The eye globes were mounted and excited by an acoustic frequency sweep from 0 to 1000 Hz, while OCT M-mode scans were collected at or around the corneal apex.

In these tests, acoustic stimulation using a frequency sweep is used to stimulate the cornea. Corneal resonance frequencies were investigated under the following conditions:
- Repeatability between different eyes with de-epithelialized corneas and a constant intraocular pressure (IOP) of 15 mmHg.
- IOP change from 15 to 30 mmHg;
- Changes in biomechanics of the cornea via crosslinking at constant IOP of 15 mmHg, measured at different coordinates around the corneal apex.

In the first test, three measurements are carried out on the porcine eye in nominal conditions (i.e. de-epithelialized cornea at constant IOP). In one measurement, no acoustic stimulation is applied to the cornea, and no substantial corneal resonance is measured. In the two measurements including stimulation, similar frequency responses are observed.

In the second test, the variation in corneal frequency response with intraocular pressure is investigated. For a single porcine eye, the IOP is varied from 15 mmHg to 30 mmHg in increments of 5 mmHg and the corneal frequency response measured at each IOP value. A definite trend of reduced corneal response with increasing IOP is observed.

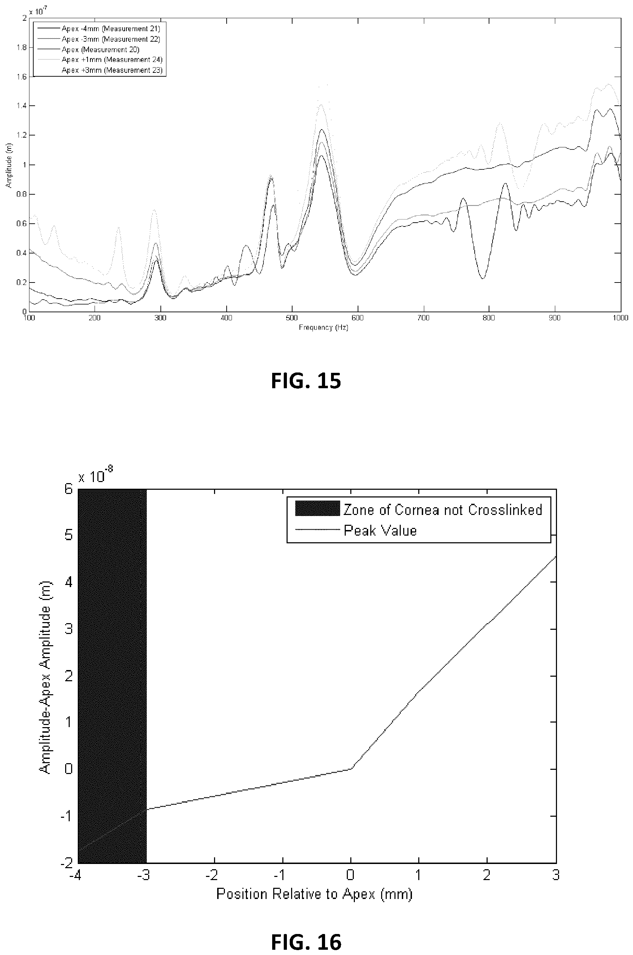

In the third test, the cornea of a porcine eye is subjected to partial corneal cross-linking. As a result, a small region of the cornea is shielded from UV radiation, creating a biomechanical inhomogeneity in the cornea. The corneal frequency response is measured at five points across the cornea, including the cross-linked region and the non-crosslinked zone. The frequency response of the cornea at these five locations is shown in Figure 15. A change in frequency response caused by difference in corneal biomechanics is clearly observed, as predicted by the simulations (and explained further below). The measurement locations are provided relative to the corneal apex. Figure 16 shows how the peak amplitude varies with measurement location. It is clear that the peak amplitude increases as the measurement moves further from the area which is not cross-linked.

As shown in these tests and results, there is significant sensitivity of the resonance modes to changes in biomechanical properties of the cornea; this can be used in differentiating keratoconic and healthy eyes, and for the evaluation and tracking of riboflavin/UV-A cornea collagen crosslinking treatment for keratoconus.

Corneal abnormality can be determined based on comparison of the frequency response amplitudes at a plurality of points across the cornea. Finite element analysis models have been completed to demonstrate the principle. Specifically, nonlinear hyper-elastic finite element models of porcine eyes were built and subjected to a modulated pressure, equal to the pressure used in the above tests. The frequency response was determined by monitoring the apex displacement over time then using Fast Fourier transformation (FFT) analysis to determine the frequency peaks. Resonance frequency and amplitudes were determined across corneal meridians for homogeneous biomechanical corneas and for corneas with local biomechanical variations.

By paying attention to the vibrations of points across the whole corneal surface, the results showed that including a keratoconic cone in the models led to significant asymmetry in resonance amplitude and some limited effect on the resonance frequency. In order to eliminate the asymmetry effect resulting from the fact that the orbital geometry is not rotationally symmetric, the asymmetry of the healthy eye models was subtracted from the asymmetry of the keratoconic eye models.

In these analyses, a distinct asymmetry is noted in the amplitude of the frequency response when a keratoconus area (called cone in clinical practice despite its edge not being circular or regular) is included in the cornea models. The corneal frequency response is monitored at a plurality o points, preferably around the corneal apex, and corneal abnormality can be detected from the asymmetry of behaviour at these points.

According to the present disclosure, frequency domain analysis is used to generate a distinctive peak amplitude variation (imbalance) map over a multitude/plurality of corneal locations over its surface, referenced by subtraction between opposing points equidistant from the apex at their peak response frequency. Then, through this map, the weak areas of the corneal surface are identified as they record higher imbalance values than the healthy areas. Through this amplitude map, the distorted area (cone location) can be detected even if the geometrical deformation is still limited.

These finite element analyses show the localisation of mechanically weakened locations of the cornea by means of corneal amplitude variation at the peak response frequency. Current cone location investigative tools for a corneal affected by a biomechanical disease (like keratoconus) rely on corneal topography or pachymetry, hence they only detect the distortion when it becomes geometrically quantifiable.

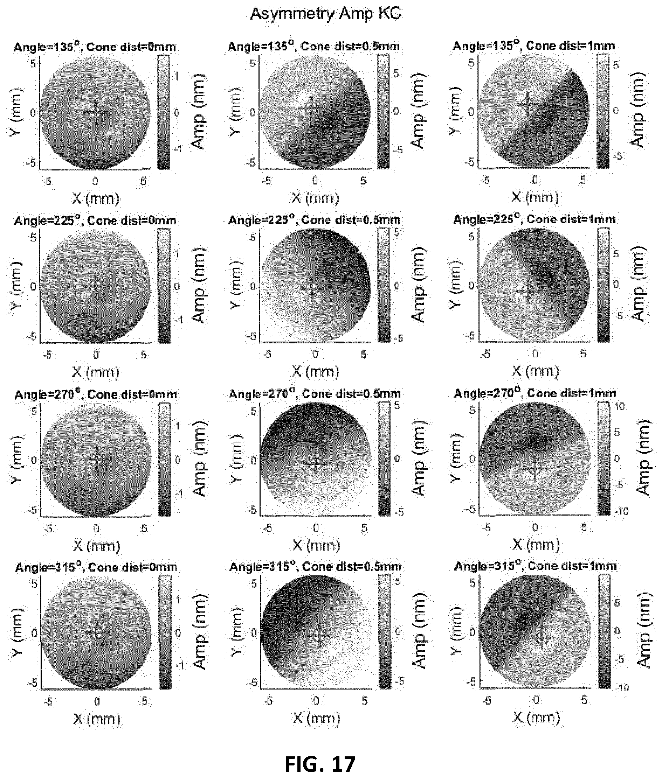

Figure 17 presents examples of the asymmetry observed in keratoconic corneas with different IOP and SSI (Stress-Strain Index, a measure of corneal stiffness). In all cases, the corneas had a keratoconus cone located in the inferior-temporal direction at 45 degrees to both the horizontal and vertical directions. The area of the cone location on the map has been circled. This figure demonstrates clearly that the ability to detect the existence of a cone is particularly strong in cases with low IOP and low SSI. However, even when IOP is 30 mmHg (above max physiologic level of 21 mmHg) and SSI is 1.5 (representing near highest stiffness corneal stiffness), the results still show evidence of irregularity.

The present disclosure also proposes the use of vibrography to determine intraocular pressure IOP. A range of finite element analysis models have been completed to demonstrate the principle. The results suggest that the IOP can be estimated using the frequency response of the apical node in both healthy and abnormal cases. Interestingly, changing the material properties of the cornea did not lead to notable changes in the detected amplitude or the resonance frequency for the same IOP. Hence, IOP can be estimated without a significant effect of corneal stiffness, which is an advantage the frequency-based analysis has over commonly-used tonometry devices in which it is hard to measure IOP without being influenced by corneal stiffness.

Using vibrography, IOP can be estimated without a significant effect of corneal stiffness, which is an advantage the frequency-based analysis has over the normal tonometry devices in which it is hard to measure the IOP without being influenced by the corneal stiffness. In vivo tests on porcine eyes show a similar trend in corneal response with change in IOP.

For both the experimental results and the finite element simulations, resonance frequencies were defined at the positions of peak amplitudes. In the present example, both showed resonance frequencies of 370 Hz. An increase of 15 mmHg in IOP resulted in a decrease of the resonance amplitude of up to 1.24±0.61 µm and a frequency shift of up to 22.7± 9.3 Hz. Biomechanical changes produced by crosslinking led to a decrease in amplitude of 2.19±0.78 µm, without significant frequency shifts. The simulations supported these trends, but showed up to 13 Hz higher frequency shifts with IOP increase. Additionally, simulations showed that localized biomechanical changes could be detected by examining asymmetries of the resonance amplitude across opposite corneal meridians. IOP and biomechanics could be decoupled, due to differential dependencies of amplitude and resonance frequency on IOP and biomechanics.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A method for screening a biomechanical abnormality of ocular tissue through deformation of the ocular tissue, the method comprising:
- generating an acoustic stimulus for delivery onto the ocular tissue in a collinear manner with an optical axis of an imaging device for producing a vibration of the ocular tissue;
- capturing with the imaging device a plurality of locations of the ocular tissue;
- processing the plurality of locations to screen the biomechanical abnormality of ocular tissue from an asymmetry in a frequency response of the vibration of the ocular tissue as measured at the plurality of locations.

2. The method of claim 1, further comprising determining a location of the biomechanical abnormality based in the frequency response of the vibration of the ocular tissue.

3. The method of any of claims 1-2, further comprising a pre-compensating process of the acoustic stimulus with a flat frequency profile.

4. The method of any of claims 1-2, further comprising a post-compensating process of the frequency response of the ocular tissue.

5. The method of any of claims 3-4, wherein the pre-compensating or the post-compensating processes comprise characterising a frequency response of the acoustic stimulus.

6. The method of claim 5, wherein characterising a frequency response of the acoustic stimulus comprises using a microphone.

7. The method of any of claims 1-6, wherein the acoustic stimulus includes an acoustic signal, and signal conditioning of the acoustic signal frequency sweep in which each frequency is probed individually and sequentially.

8. The method of any of claims 1-6, wherein the acoustic stimulus includes an acoustic signal, and sinc regime where a range of frequencies are probed simultaneously using a sinc pulse.

9. The method of any of claims 1-8, further comprising obtaining intraocular pressure of the ocular tissue from a resonance mode analysis of the vibration of the ocular tissue.

10. A system for screening a biomechanical abnormality of ocular tissue (C), the system comprising:
- an acoustic stimulation module (20) configured to deliver at least one acoustic wave (21) onto the ocular tissue and to produce a vibration thereof;
- an imaging device (10) operatively coupled to the acoustic stimulation module (20);
**characterised in that**
- the acoustic stimulation module (20) comprises a hole (22), the hole configured to be aligned with an optical axis of the imaging device, such that the acoustic wave delivered onto the ocular tissue can be made collinear with the optical axis of the imaging device;
- the imaging device (100, 100') is configured to capture a plurality of locations of the ocular tissue;
the system further comprising:
- processing means configured to process the plurality of locations provided by the imaging device to screen the biomechanical abnormality of ocular tissue from an asymmetry in a frequency response of the vibration of the ocular tissue as measured at the plurality of locations.

11. The system of claim 10, wherein the processing means are adapted configured to carry out finite-element-model based calculation for screening the biomechanical abnormality of the ocular tissue.

12. The system of any of claims 10-11, wherein the processing means are adapted configured to carry out finite-element-model based calculation for obtaining an intraocular pressure of the ocular tissue.

13. The system of any of claims 10-12, wherein the finite-element-model based calculation is carried out from resonance mode analysis.

14. A method for screening a biomechanical abnormality of ocular tissue through deformation of the ocular tissue, wherein the method is carried out with the system of any of claims 10-13.
